# EUROPEAN PATENT APPLICATION

(11) **EP 4 434 964 A1**
(43) Date of publication of application: **25.09.2024**
(21) Application number: 22895596.9
(22) Date of filing: 15.11.2022
(51) Int. Cl.: C07C 233/10, C07B 61/00, C07C 233/25

(54) **METHOD FOR PRODUCING ACETAMINOPHEN**

(30) Priority: 16.11.2021 JP 2021186485
(71) Applicant: API Corporation, Chikujo-gun, Fukuoka 871-0801 (JP)
(72) Inventor: KOBA, Yurie, Chikujo-gun, Fukuoka 871-0801 (JP); TANIIKE, Hirotsugu, Chikujo-gun, Fukuoka 871-0801 (JP); WATANABE, Naoyuki, Chikujo-gun, Fukuoka 871-0801 (JP)
(74) Representative: Godemeyer Blum Lenze Patentanwälte Partnerschaft mbB - werkpatent
(86) International application number: PCT/JP2022/042371
(87) International publication number: WO 2023/090315

(57) **Abstract**

A method for producing acetaminophen, the method comprising causing p-nitrophenol to undergo an acetamination reaction to produce the acetaminophen, by passing a solution containing the p-nitrophenol through a column packed with a catalyst while also passing an acetylating agent and hydrogen through the column. The catalyst is a supported metal catalyst in which a metal element is supported on a monolith porous body, and a reaction temperature of the acetamination reaction is 0°C to 60°C, and a reaction pressure of the acetamination reaction is 0.1 MPa to 1 MPa.

## Description

### Technical Field

The present invention relates to a method for producing acetaminophen, which is useful as a medicine.

### Background Art

Acetaminophen is an antipyretic and analgesic drug that has been widely used for a long time. Acetaminophen is a safe drug that can be administered not only to adults but also to children.

In the related art, known methods for producing acetaminophen include batch reaction methods. For example, one known method is a method in which p-nitrophenol, acetic acid, and a metal catalyst are added to a reaction vessel, hydrogen is added thereto, and a reaction is caused to take place at a high temperature to produce acetaminophen (Patent Literature 1).

Unfortunately, in the method of Patent Literature 1, since the reaction temperature is high, and intense heat generation occurs when the catalyst is added, control of the reaction is difficult.

Accordingly, there is a need for an industrial production method that is safer and highly productive.

A method that increases productivity is a continuous reaction method.

For example, a method is known in which p-nitrophenol is added to an acetic anhydride/acetic acid solution to form a solution, and the solution is passed through a column packed with a noble metal catalyst, specifically, a Pd/C catalyst, to cause the p-nitrophenol to undergo a reaction at a hydrogen pressure of 8 MPa to 10 MPa and a reaction temperature of 90 to 140°C, thereby continuously producing acetaminophen (Patent Literature 2).

Unfortunately, in the method of Patent Literature 2, equipment that can withstand very high pressure conditions is required, and the reaction temperature is high. In addition, in instances where the reaction takes place continuously at a high temperature and a high pressure for a long time, there is a possibility that early degradation of the catalyst may occur.

Accordingly, there is a need for a production method that is a continuous production method that enables reactions to take place under milder conditions, conserves energy, and can be implemented at low costs, with the costs including, for example, equipment cost.

For the pore size, through hole size, and particle size of the support, it is known that the skeleton of each particle of a particulate porous material has a three-dimensional continuous network structure in a two-level hierarchically porous structure when the mode size in the size distribution for the through holes is greater than or equal to 5 times the mode size of the pores and when the particle size is greater than or equal to 5 times the mode size of the through holes (Patent Literature 3). In this Patent Literature 3, however, there is no description of a support for supported metal catalysts for acetaminophen production.

### Citation List

### Patent Literature

PTL 1: WO 2017/154024 A1
PTL 2: CN 102060729 A
PTL 3: JP 6501282 B

### Summary of Invention

### Technical Problem

An object of the present invention is to provide a method for continuously producing acetaminophen safely and inexpensively with high selectivity and good yield, at a low reaction temperature and a low reaction pressure.

The present inventors discovered that acetaminophen can be produced safely and inexpensively with high selectivity and good yield, even at a low reaction pressure and a low reaction temperature, by causing p-nitrophenol to undergo a reaction by continuously passing a solution containing the p-nitrophenol through a column packed with a catalyst in which a metal element is supported on a monolith porous body, while also continuously passing an acetylating agent and hydrogen through the column.

Features of the present invention are as follows.

[1] A method for producing acetaminophen, the method comprising causing p-nitrophenol to undergo an acetamination reaction to produce the acetaminophen, by passing a solution containing the p-nitrophenol through a column packed with a catalyst while also passing an acetylating agent and hydrogen through the column, wherein
   the catalyst is a supported metal catalyst in which a metal element is supported on a monolith porous body, and
   a reaction temperature of the acetamination reaction is 0°C to 60°C, and a reaction pressure of the acetamination reaction is 0.1 MPa to 1 MPa.
[2] The method for producing acetaminophen according to [1], wherein the monolith porous body is an inorganic monolith porous body.
[3] The method for producing acetaminophen according to [1] or [2], wherein the monolith porous body has a skeleton formed of an inorganic compound in a three-dimensional continuous network structure and is in a two-level hierarchically porous structure having through holes created in gaps in the skeleton and pores extending inward from a surface of the skeleton and created dispersedly on the surface.
[4] The method for producing acetaminophen according to any one of [1] to [3], wherein the monolith porous body is a skeleton formed of an inorganic compound in a three-dimensional continuous network structure, and the metal element is palladium and/or platinum.
[5] The method for producing acetaminophen according to [3] or [4], wherein the monolith porous body is a monolith porous body in which a mode size in a size distribution for the pores is greater than or equal to 1 nm and less than or equal to 100 nm.
[6] The method for producing acetaminophen according to any one of [3] to [5], wherein a mode size in a size distribution for the through holes in the monolith porous body is greater than or equal to 5 times a mode size of the pores and is greater than or equal to 0.1 um and less than or equal to 50 um.
[7] The method for producing acetaminophen according to any one of [3] to [6], wherein the monolith porous body is in form of particles and has a particle size that is greater than or equal to twice a mode size of the through holes and is greater than or equal to 20 um.
[8] The method for producing acetaminophen according to any one of [1] to [7], wherein an amount of the supported metal element in the supported metal catalyst is 0.1 mass% to 10 mass% based on a mass of the supported metal catalyst. Advantageous Effects of Invention

With the method of the present invention for producing acetaminophen, it is possible to continuously produce acetaminophen safely and inexpensively with high selectivity and good yield, at a low reaction temperature and a low reaction pressure.

### Brief Description of Drawings

[Fig. 1] Fig. 1 is a system diagram of a flow synthesis system, illustrating an exemplary embodiment of a method of the present invention for producing acetaminophen.
[Fig. 2] Fig. 2 is a system diagram of a flow synthesis system including a back-pressure valve, illustrating another exemplary embodiment of the method of the present invention for producing acetaminophen.

### Description of Embodiments

The present invention will be described in detail below.

### [Method for producing acetaminophen]

The method for producing acetaminophen of the present invention comprises causing p-nitrophenol to undergo an acetamination reaction to produce the acetaminophen, by passing a solution containing the p-nitrophenol (This solution may be referred to as "p-nitrophenol solution" hereinafter.) through a column packed with a catalyst while also passing an acetylating agent and hydrogen through the column, (This step may be referred to as "acetamination step of the present invention" hereinafter.) wherein the catalyst is a supported metal catalyst in which a metal element is supported on a monolith porous body (The monolith porous body may be referred to as " monolith porous body of the present invention" hereinafter.) (The supported metal catalyst may be referred to as "supported metal catalyst of the present invention" hereinafter.), and a reaction temperature of the acetamination reaction is 0°C to 60°C, and a reaction pressure of the acetamination reaction is 0.1 MPa to 1 MPa.

### <Acetamination Step>

Methods for implementing the acetamination step of the present invention are not particularly limited.

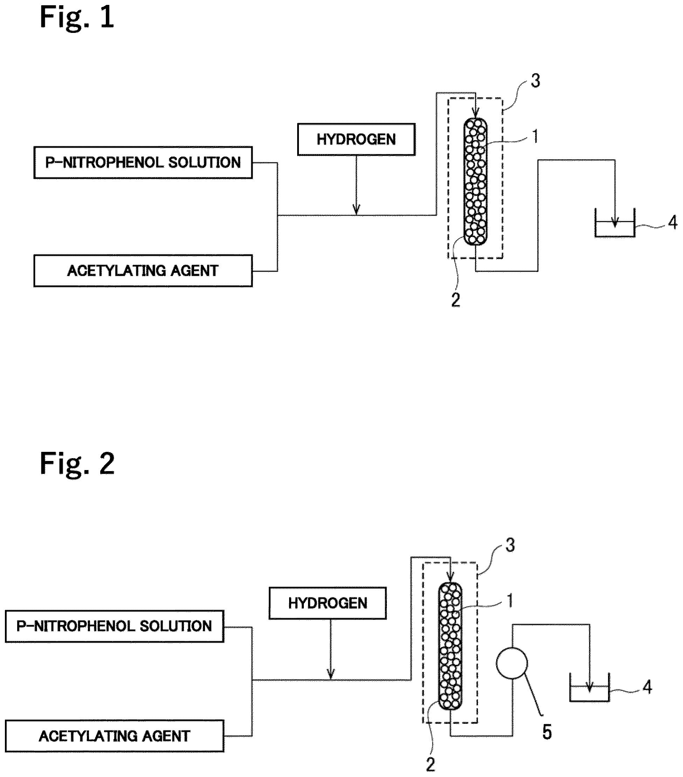

An exemplary method is a method that uses a flow synthesis system, as illustrated in Figs. 1 and 2. In the method, a p-nitrophenol solution is continuously passed through a reaction vessel 3, which includes a column 2 packed with a supported metal catalyst 1 of the present invention, while an acetylating agent and hydrogen are also continuously passed through the reaction vessel 3, to cause the p-nitrophenol to continuously undergo an acetamination reaction with the acetylating agent and the hydrogen in the presence of the supported metal catalyst of the present invention within the column 2, and a reaction product liquid containing acetaminophen, which flows from the column 2, is received in a collection reservoir 4.

The flow synthesis system of Fig. 2 has a similar configuration to that of the flow synthesis system of Fig. 1, with a difference being that a back-pressure valve 5 is provided in a flow path through which the reaction product liquid coming from the reaction vessel 3 is delivered to the collection reservoir 4.

This flow synthesis system will be described later.

### <P-nitrophenol Solution>

The p-nitrophenol, which is a raw material for the production of acetaminophen, may be a commercially available product or one prepared in accordance with a known method.

The solvent for use in the p-nitrophenol solution is not particularly limited as long as the solvent can dissolve p-nitrophenol and does not retard the progress of the reaction. Examples of the solvent include alcohol solvents and carboxylic acid solvents. Examples of the alcohol include aliphatic alcohol solvents having 1 to 4 carbon atoms such as methanol, ethanol, and propanol, and examples of the carboxylic acid include aliphatic carboxylic acid solvents having 1 to 3 carbon atoms such as formic acid, acetic acid, and propionic acid. From the viewpoint of cost, reactivity, and the like, aliphatic carboxylic acid solvents having 1 to 3 carbon atoms are preferred, and acetic acid is particularly preferred.

One of these solvents may be used alone, or two or more thereof may be combined in any combination in any ratio and used. From the viewpoint of ease of solvent removal, it is preferable to use one kind of solvent alone.

A concentration of the p-nitrophenol in the p-nitrophenol solution is not particularly limited as long as the flow thereof into the column is not hindered. The concentration of the p-nitrophenol in the p-nitrophenol solution may be specified from the standpoint of productivity and reactivity and is typically 0.1 mass% to 50 mass%, preferably 5 mass% to 40 mass%, and particularly preferably 10 mass% to 30 mass%.

### <Hydrogen>

An amount of use of the hydrogen (hydrogen gas) is not particularly limited as long as the reaction can proceed. The amount of use of the hydrogen (hydrogen gas) is typically greater than or equal to 1 mol and preferably greater than or equal to 3 mol and is typically less than or equal to 20 mol and preferably less than or equal to 10 mol, per mol of the p-nitrophenol.

Methods for feeding the hydrogen are not particularly limited. The hydrogen may be continuously introduced into and mixed with the p-nitrophenol solution or a p-nitrophenol solution that contains an acetylating agent, in a flow path upstream of the column 2, or the hydrogen may be directly injected into the column 2. The hydrogen may be used by being partially or entirely dissolved in the solvent of the p-nitrophenol solution. It is preferable to use hydrogen in the above-mentioned amount by sufficiently mixing with the solvent before the p-nitrophenol solution passes through the column 2.

Although hydrogen may be used by being mixed with an inert gas such as nitrogen, helium, or argon, it is preferable to use hydrogen alone.

### <Acetylating Agent>

The acetylating agent is not particularly limited as long as the acetylating agent can acetylate amino groups. Typically, the acetylating agent to be used is one or more of acetylating agents such as acetic anhydride, acetyl chloride, and the like. Acetic anhydride is preferable from the standpoint of cost and reactivity.

An amount of use of the acetylating agent is not particularly limited. The amount of use of the acetylating agent may be specified from the standpoint of cost and reactivity and is typically 1 mol to 10 mol, preferably 1 mol to 5 mol, and more preferably 1 mol to 2 mol, per mol of the p-nitrophenol.

The acetylating agent may be premixed with the solution containing p-nitrophenol, may be continuously mixed with the p-nitrophenol solution by introducing the acetylating agent into at least one of the p-nitrophenol solution delivery flow paths upstream and downstream of the column 2, or may be introduced into the column 2 separately from the p-nitrophenol solution and continuously mixed with the p-nitrophenol solution within the column 2. From the standpoint of rapidly converting an unstable intermediate product into a target product, it is preferable that the acetylating agent be continuously mixed with the p-nitrophenol solution in the flow path upstream of the column 2.

### <Supported Metal Catalyst>

The supported metal catalyst of the present invention is a catalyst in which a metal is fixed, with the metal element being supported on a carrier.

The metal element that can be used in the supported metal catalyst of the present invention is not particularly limited as long as the metal element has activity for reducing nitro groups. Typically, the metal element may be palladium (Pd), platinum (Pt), rhodium (Rh), ruthenium (Ru), silver (Ag), or a mixture of two or more of these. Among these metal elements, Pd alone and a mixture of Pd and at least one selected from Pt, Rh, Ru, and Ag are preferable. From the standpoint of catalytic performance, Pd and/or Pt are preferable, and Pd alone is particularly preferable.

An amount of the supported metal element may be specified from the standpoint of catalytic performance and cost. The lower limit of a content of the metal element in the supported metal catalyst of the present invention is typically greater than or equal to 0.1 mass%, preferably greater than or equal to 0.3 mass%, and more preferably greater than or equal to 0.5 mass%, and the upper limit of the content is typically less than or equal to 10 mass% and preferably less than or equal to 7 mass%.

### <Support>

In the present invention, a support is a substance that does not inhibit reaction and serves as a foundation on which a metal element is fixed.

The structure of the support in the present invention is typically a monolith porous body, preferably an inorganic monolith porous body.

A monolith porous body has a high porosity and a large specific surface area and, therefore, enables the passage and reaction of solution to proceed efficiently. Furthermore, a monolith porous body has through holes in the order of micrometers inside its particles, which allow solution to diffuse rapidly to the deep inside of the particles. With a monolith porous body, therefore, the resistance of flow paths can be reduced, and it is possible to improve reactivity and productivity, compared with a porous material in a single-pore structure having no through holes.

It is preferable that each particle of the monolith porous body of the present invention have a skeleton in a three-dimensional continuous network structure and, furthermore, have a structure having through holes created in gaps in the skeleton and pores extending inward from the surface of the skeleton and created dispersedly on the surface (This structure may be referred to as "two-level hierarchically porous structure" hereinafter.).

Preferably, the structure of the monolith porous body of the present invention is a bicontinuous structure having through holes and pores with different sizes as the two levels.

Examples of the constituent of the monolith porous body for use in the present invention include inorganic compounds, such as silica gel or titanium oxide, and organic compounds, such as epoxy resins. For use as the constituent of the monolith porous body, inorganic compounds are preferable in terms of cost and chemical stability. Silica gel is particularly preferable.

The form of the monolith porous body for use in the present invention can be masses, particles, or powder. From the standpoint of reducing the resistance of flow paths and improving reactivity, it is preferable that the monolith porous body be in the form of particles.

As mentioned above, for the pore size, through hole size, and particle size of a support, it is known that the skeleton of each particle of a particulate porous material has a three-dimensional continuous network structure in a two-level hierarchically porous structure when the mode size in the size distribution for the through holes is greater than or equal to 5 times the mode size of the pores and when the particle size is greater than or equal to 5 times the mode size of the through holes (Patent Literature 3).

For the monolith porous body of the present invention, it is preferable, from the standpoint of productivity, that the mode size in the size distribution for the pores be greater than or equal to 1 nm and less than or equal to 100 nm. More preferably, this mode size is greater than or equal to 2 nm and less than or equal to 20 nm.

It is preferable that the monolith porous body of the present invention be a porous support having such relatively large pores.

For the monolith porous body of the present invention, it is preferable, from the standpoint of productivity, that the mode size in the size distribution for the through holes be greater than or equal to 5 times, preferably 5 times to 100 times, 5 times to 50 times in particular, the mode size of the pores.

For the monolith porous body of the present invention, it is preferable, from the standpoint of reactivity and productivity, that the mode size in the size distribution for the through holes be greater than or equal to 0.1 um and less than or equal to 50 um. More preferably, this mode size is greater than or equal to 0.1 um and less than or equal to 30 um.

It is preferable that the monolith porous body of the present invention be in the form of particles and have a particle size that is greater than or equal to twice the mode size of the through holes. From the standpoint of reactivity and productivity, it is preferable that the particle size be 1000 times to 5000 times the mode size of the through holes.

The particle size of the monolith porous body of the present invention in the form of particles is typically 20 um to 500 um, preferably 50 um to 500 um, and particularly preferably 50 um to 250 um. When the particle size of the monolith porous body is too small, an increase in the resistance of flow paths may cause a decrease in productivity, and when the particle size of the monolith porous body is too large, reactivity may decrease.

In the present invention, the respective mode sizes of the pores and through holes in the monolith porous body can be measured with a mercury intrusion method or nitrogen gas adsorption method in accordance with a common procedure.

The particle size of the monolith porous body is a particle size based on the mesh size of the sieve used for the classification treatment.

The monolith porous body for use in the present invention may be a commercially available product, examples of which include DualPore, manufactured by DPS Inc. ("DualPore" is a registered trademark), MonoTrap, manufactured by GL Sciences Inc. ("MonoTrap" is a registered trademark), and MonoPure, manufactured by Kiko Tech Co., Ltd.

Preferably, the supported metal catalyst of the present invention is a catalyst in which Pd is supported on a monolith porous body based on a skeleton formed of a compound in a three-dimensional continuous network structure (which may hereinafter be referred to as "Pd/DualPore (registered trademark)") or a catalyst in which Pt is supported on a monolith porous body based on a skeleton formed of a compound in a three-dimensional continuous network structure (which may hereinafter be referred to as "Pt/DualPore (registered trademark)"). Particularly preferably, the supported metal catalyst is Pd/DualPore (registered trademark).

With the use of the supported metal catalyst of the present invention, it is possible to produce acetaminophen highly efficiently, safely, and inexpensively with high selectivity and good yield, even at a low pressure and a low temperature.

The supported metal catalyst of the present invention can be produced with methods known in the art, such as the method described in Yamada, T. et al. Catal. Sci. Technol. 2020, 10, 6359. For example, the supported metal catalyst can be produced as follows. The monolith porous body and a metal salt are added to an organic solvent. The metal is reduced subsequent to thorough stirring, and the resulting monolith porous body on which the metal has been adsorbed is collected by filtration, washed with water and methanol, and dried.

### <Flow Synthesis System>

The flow synthesis system, which is suitable for the implementation of the method of the present invention for producing acetaminophen, is a system that uses a reaction vessel having an inlet and an outlet and simultaneously carries out the addition of raw materials through the inlet, the reaction, and the collection of the formed product from the outlet. The concept of the flow synthesis system is well known to those skilled in the art (e.g., "Flow-Micro Synthesis", published by Kagaku Dojin in 2014, page 9). In the flow synthesis system, the column into which the supported metal catalyst of the present invention is packed is in the form of a narrow pipe.

The material of the column associated with the present invention is not particularly limited. Examples of the material of the column include glass, stainless steel (SUS), Hastelloy, and Teflon (registered trademark). Preferably, the material is SUS or Hastelloy.

The column may have a size that is not particularly limited as long as the size is suitable for the reaction. Examples of columns that may be used include columns having a size of 10 mm (diameter) × 100 mm (length) and columns having a size of 10 mm (diameter) × 250 mm (length).

The shape of the column may be spiral or annular, but typically, the column is a straight pipe.

The cross-sectional shape of the column is typically round or rectangular, but preferably is round. It is, therefore, preferable that the column be in a cylindrical shape.

The number of columns is not particularly limited, but industrially, it is typically 1 to 10000.

The column may be one in which a column reaction zone has been created by joining or combining multiple column-forming members.

Examples of catalyst-packed columns include one in which Pd/DualPore (registered trademark) (Pd: 0.39 g, 0.04 mmol/g, DualPore (registered trademark)) is close-packed into an SUS column (4.6 mm × 100 mm) and one in which Pd/DualPore (registered trademark) (Pd: 0.39 g, 0.09 mmol/g, DualPore (registered trademark)) is close-packed into an SUS column (4.6 mm × 100 mm) .

A tube that is used as the flow path for introducing and discharging a substrate and the like into and from the column is not particularly limited. Specific examples of the tube include a Teflon tube having an inside diameter of 1 mm ("Teflon" is a registered trademark).

The introduction and discharge of the substrate and the like into the column can be carried out by delivering the liquid with a syringe pump, a diaphragm pump, a mass flow controller, and the like.

A back-pressure valve and an in-line analyzer may be provided in the flow path on the side to which the reaction product liquid from the column is discharged.

### <Reaction Conditions>

The reaction temperature of the acetamination reaction of the present invention is a temperature of the outside of the column packed with the supported metal catalyst of the present invention. The reaction temperature may be specified from the standpoint of reactivity, productivity, and the like and is typically 0°C to 60°C, preferably 5°C to 50°C, and particularly preferably 10°C to 40°C. When the reaction temperature is less than any of the lower limits, the reactivity may decrease. When the reaction temperature is greater than any of the upper limits, a side reaction may cause a decrease in the yield and purity and degradation in the supported metal catalyst of the present invention.

The lower limit of a reaction pressure of the acetamination reaction of the present invention is typically greater than or equal to 0.1 MPa and preferably greater than or equal to 0.2 MPa, and the upper limit thereof is typically less than or equal to 1 MPa, preferably less than or equal to 0.8 MPa, and particularly preferably less than or equal to 0.6 MPa. In instances where the reaction is performed at a reaction pressure within any of these ranges, a hydrogen concentration of the p-nitrophenol solution increases, which enables the reaction to proceed efficiently.

The reaction pressure can be adjusted by applying a back pressure with a back-pressure valve or the like to the flow path downstream of the column packed with the supported metal catalyst of the present invention.

A reaction time of the acetamination reaction of the present invention is the time (retention time) during which the reaction liquid remains within the column packed with the supported metal catalyst of the present invention. The reaction time is typically 1 second to 60 seconds, depending on the reaction temperature and the reaction pressure.

A space velocity (S/V) is the amount of solution of p-nitrophenol passing through the catalyst per unit time divided by the column volume. From the viewpoint of productivity, the space velocity (S/V) is usually 100 h⁻¹ to 1000 h⁻¹, preferably 120 h⁻¹ to 700 h⁻¹, and particularly preferably 140 h⁻¹ to 400 h⁻¹.

### <Post-treatment>

The isolation of acetaminophen, which is the target product, from the reaction product liquid obtained in the acetamination step of the present invention, may be carried out by performing, on the reaction product liquid, a process such as depressurization, neutralization, liquid-phase separation, condensation, or filtration or by using a known purification method, such as crystallization or column chromatography.

### EXAMPLES

The present invention will be described in more detail with reference to examples. The scope of the present invention is not limited to the examples described below.

In the following Examples and Comparative Examples, a ratio between the p-nitrophenol and acetic anhydride is 1:1.2 unless otherwise specified.

### [Abbreviations]

In the examples, abbreviations each represent a compound, as listed below.
PAP: p-aminophenol
APAP: acetaminophen
PAAPA: 4-acetamidophenyl acetate
PNP: p-nitrophenol
PNPA: 4-nitrophenyl acetate
AcOH: acetic acid
MeOH: methanol
PAP, PAAPA and PNPA are by-products.

### [Flow Synthesis Apparatus]

The following flow synthesis apparatus was used in the Examples and Comparative Examples, described below.

Asia Flow Chemistry System, manufactured by Syriss Ltd.

### [Analysis Method 1 (HPLC)]

The apparatus and conditions used for the analysis of the reaction product liquid in the Examples and Comparative Examples, described below, are as shown in Table 1 below.

**[Table 1]**

| Analyzer | Agilent 1100, manufactured by Agilent | | |
|---|---|---|---|
| Column | Unison UK-C18 | | |
| | 3 µm, 4.6 mm I.D.×250 mm | | |
| Mobile phase A | 0.1 vol% aqueous phosphoric acid solution | | |
| Mobile phase B | Acetonitrile | | |

| Gradient | Time (min) | Mobile phase A (%) | Mobile phase B (%) |
|---|---|---|---|
| | 0 | 90 | 10 |
| | 0 to 15 | 90→20 | 10→80 |
| Flow rate | 1.0 mL/min | | |
| Detection wavelength | 240 nm | | |
| Column temperature | 40°C | | |
| Analysis time | 15 min | | |
| Retention time | PAP: 2.7 min | | |
| | APAP: 6.3 min | | |
| | PAAPA: 9.7 min | | |
| | PNP: 11.5 min | | |
| | PNPA: 13.8 min | | |

### [Supported Metal Catalysts]

The supported metal catalysts used in the Examples and Comparative Examples, described below, are as shown in Table 2 below. In Table 2, "TM" represents "(registered trademark)." The constituents of the supports in catalysts 1 to 7 in Table 2 are as follows.
Catalysts 1 to 5: Silica gel
Catalyst 6: Activated carbon
Catalyst 7: A styrene-divinylbenzene synthetic adsorbent

**[Table 2]**

| | Supported metal catalyst | Support structure | Pd content [mass%] | Support particle size [µm] |
|---|---|---|---|---|
| Catalyst 1 | Pd/DualPore^{™} (1) | Two-level hierarchically porous structure | 0.39 | 63 to 212 |
| Catalyst 2 | Pd/DualPore^{™} (2) | Two-level hierarchically porous structure | 0.85 | 63 to 212 |
| Catalyst 3 | Pd/DualPore^{™} (3) | Two-level hierarchically porous structure | 0.96 | 63 to 212 |
| Catalyst 4 | Pd/DualPore^{™} (4) | Two-level hierarchically porous structure | 1.10 | 63 to 212 |
| Catalyst 5 | Pd/DualPore^{™} (5) | Two-level hierarchically porous structure | 0.95 | 212 to 500 |
| Catalyst 6 | 5% Pd/C (beads) | Single-pore structure | 5 | larger than 200 |
| Catalyst 7 | 10% Pd/DIAION^{™} HP20 | Single-pore structure | 10 | larger than 250 |

### [Example 1]

Acetaminophen was synthesized in the flow synthesis system, illustrated in Fig. 2. The reaction vessel used was one in which catalyst 1 (Pd/DualPore (registered trademark) (1)) was packed into an SUS column having a size of 4.6 mm (inside diameter) × 100 mm (length).

1 L of a solution mixture of an acetic acid solution of p-nitrophenol (concentration: 0.84 mol/L) and 103 g of acetic anhydride (1.2 mol per mol of the p-nitrophenol) (hereinafter, p-nitrophenol solution) was continuously mixed with hydrogen gas (4.9 mol per mol of the p-nitrophenol) in a flow path upstream of the column 2, and the mixture was flowed through the reaction vessel 3 for 20 minutes. The obtained reaction product liquid was analyzed with the analysis method 1, and the results are shown in Table 3. The obtained reaction product liquid was found to contain acetaminophen (0.78 mol/L, yield: 94.0%). In Table 3, "S/V" is space velocity (h⁻¹), the volume of the p-nitrophenol solution that passes through the catalyst per unit time divided by the volume of the column.

The feed rate of the p-nitrophenol solution was maintained at 4.5 mL/minute with a diaphragm pump and a cylinder pump. The feed rate of the hydrogen gas was maintained at 375 mL/minute with a mass flow controller. The reaction vessel was maintained at 30°C in a water bath.

### [Examples 2 to 6]

A reaction was performed as in Example 1, except that as opposed to Example 1, the supported metal catalyst, the feed rate of the p-nitrophenol solution, and the number of equivalents of hydrogen were as shown in Table 3. The obtained reaction product liquid was analyzed as in Example 1, and the results are summarized in Table 3.

### [Comparative Example 1]

A reaction was performed as in Example 1, except that as opposed to Example 1, catalyst 1 (Pd/DualPore (registered trademark) (1)) and the acetic acid solution of p-nitrophenol were changed to catalyst 6 (5% Pd/C (beads) (manufactured by N.E. Chemcat Corporation)) and a methanol solution of p-nitrophenol (concentration: 0.84 mol/L), respectively, and the feed rate of the solution, the number of equivalents of hydrogen (the number of moles of hydrogen per mol of the p-nitrophenol), and the reaction pressure (back pressure) were as shown in Table 3. The obtained reaction product liquid was analyzed as in Example 1, and the results are summarized in Table 3.

Note that using 5% Pd/carbon (powder) for comparative examples was considered, but Pd/carbon (beads) was used in the Comparative Examples, because it was surmised that, since Pd/carbon (powder) has a very small particle size, which causes a very high pressure loss, the solution of p-nitrophenol could not be passed through the reaction vessel, and, therefore, the reaction would not proceed, unless a high pressure was used.

### [Example 7]

A reaction was performed as in Example 1, except that as opposed to Example 1, catalyst 1 (Pd/DualPore (registered trademark) (1)) was changed to catalyst 5 (Pd/DualPore (registered trademark) (5)). The obtained reaction product liquid was analyzed as in Example 1, and it was found that S/V was 163/h.

### [Comparative Example 2]

A reaction was performed as in Example 1, except that as opposed to Example 1, catalyst 1 (Pd/DualPore (registered trademark) (1)) and the acetic acid solution of p-nitrophenol were changed to catalyst 7 (10% Pd/DIAION (registered trademark) HP20 (manufactured by Mitsubishi Chemical Corporation)) and a methanol solution of p-nitrophenol (concentration: 0.84 mol/L), respectively, and the feed rate of the solution was changed to 4 mL/minute, the number of equivalents of hydrogen was changed to 3.6 MR, and the reaction pressure (back pressure) was changed to 0.5 MPa. The obtained reaction product liquid was analyzed as in Example 1, and it was found that S/V was 30/h.

**[Table 3]**

| | Supported metal catalyst | Pd content [mass%] | Support particle size [µm] | Solvent | PNP solution feed rate [mL/min] | Hydrogen equivalents [MR] | Reaction pressure [MPa] | S/V [1/h] | Results of analysis of reaction product liquid (yield) | | | | |
|---|---|---|---|---|---|---|---|---|---|---|---|---|---|
| | | | | | | | | | PNP [%] | APAP [%] | PAAPA [%] | PNPA [%] | PAP [%] |
| Example 1 | Catalyst 1 | 0.39 | 63 to 212 | AcOH | 4.5 | 4.9 | 0.1 | 163 | 5.8 | 94.0 | 0.1 | 0 | 0 |
| Example 2 | Catalyst 2 | 0.85 | 63 to 212 | AcOH | 4.5 | 4.9 | 0.1 | 163 | 1.8 | 98.1 | 0.1 | 0 | 0 |
| Example 3 | Catalyst 3 | 0.96 | 63 to 212 | AcOH | 4.5 | 4.9 | 0.1 | 163 | 0.8 | 99.0 | 0.2 | 0 | 0 |
| Example 4 | Catalyst 3 | 0.96 | 63 to 212 | AcOH | 4.5 | 3.3 | 0.1 | 163 | 10.5 | 89.3 | 0.2 | 0 | 0 |
| Example 5 | Catalyst 3 | 0.96 | 63 to 212 | AcOH | 5.5 | 3.3 | 0.1 | 199 | 9.4 | 90.4 | 0.2 | 0 | 0 |
| Example 6 | Catalyst 4 | 11 | 63 to 212 | AcOH | 4.5 | 4.9 | 0.1 | 163 | 0.0 | 99.6 | 0.4 | 0 | 0 |
| Comparative Example 1 | Catalyst 6 | 5 | 200 < | MeOH | 1 | 3.6 | 0.5 | 8 | 16.3 | 82.7 | 1.0 | 0 | 0 |

Examples 1 to 7 and Comparative Examples 1 and 2 demonstrate that in the instance where a monolith porous body is used, the space velocity (S/V) increases, and productivity can be improved.

Examples 1 to 6 and Comparative Example 1 demonstrate that in the instance where a monolith porous body is used, acetaminophen can be produced efficiently with a smaller amount of supported metal, in a shorter reaction time, and with higher selectivity and better yield, than with a support used in the related art.

Examples 1 to 3 and 6 demonstrate that in the instance where the amount of supported Pd is increased, acetaminophen can be produced efficiently with better yield.

Examples 4 and 5 demonstrate that even when the number of equivalents of hydrogen is reduced to a value close to 3 equivalents, which is the minimum number of equivalents required for the reaction, acetaminophen can be produced efficiently with high selectivity and good yield.

### Industrial Applicability

The method of the present invention for producing acetaminophen can continuously produce acetaminophen, which is useful as a medicine, from p-nitrophenol, safely and inexpensively, with high selectivity and good yield, under mild conditions of a low reaction temperature and a low reaction pressure, without requiring high-pressure reaction equipment, and, therefore, the method is industrially useful.

Although the present invention has been described in detail with reference to particular embodiments, it will be apparent to those skilled in the art that various changes can be made without departing from the spirit and the scope of the present invention.

The present application is based on Japanese Patent Application No. 2021-186485 filed on November 16, 2021, which is herein incorporated in its entirety by reference. Reference Signs List

- 1: Supported metal catalyst of present invention
- 2: Column
- 3: Reaction vessel
- 4: Collection reservoir
- 5: Back-pressure valve

## Claims

1. A method for producing acetaminophen, the method comprising causing p-nitrophenol to undergo an acetamination reaction to produce the acetaminophen, by passing a solution containing the p-nitrophenol through a column packed with a catalyst while also passing an acetylating agent and hydrogen through the column, wherein
the catalyst is a supported metal catalyst in which a metal element is supported on a monolith porous body, and
a reaction temperature of the acetamination reaction is 0°C to 60°C, and a reaction pressure of the acetamination reaction is 0.1 MPa to 1 MPa.

2. The method for producing acetaminophen according to Claim 1, wherein the monolith porous body is an inorganic monolith porous body.

3. The method for producing acetaminophen according to Claim 1 or 2, wherein the monolith porous body has a skeleton formed of an inorganic compound in a three-dimensional continuous network structure and is in a two-level hierarchically porous structure having through holes created in gaps in the skeleton and pores extending inward from a surface of the skeleton and created dispersedly on the surface.

4. The method for producing acetaminophen according to any one of Claims 1 to 3, wherein the monolith porous body is a skeleton formed of an inorganic compound in a three-dimensional continuous network structure, and the metal element is palladium and/or platinum.

5. The method for producing acetaminophen according to Claim 3 or 4, wherein the monolith porous body is a monolith porous body in which a mode size in a size distribution for the pores is greater than or equal to 1 nm and less than or equal to 100 nm.

6. The method for producing acetaminophen according to any one of Claims 3 to 5, wherein a mode size in a size distribution for the through holes in the monolith porous body is greater than or equal to 5 times a mode size of the pores and is greater than or equal to 0.1 um and less than or equal to 50 um.

7. The method for producing acetaminophen according to any one of Claims 3 to 6, wherein the monolith porous body is in form of particles and has a particle size that is greater than or equal to twice a mode size of the through holes and is greater than or equal to 20 um.

8. The method for producing acetaminophen according to any one of Claims 1 to 7, wherein an amount of the supported metal element in the supported metal catalyst is 0.1 mass% to 10 mass% based on a mass of the supported metal catalyst.
